# EUROPEAN PATENT APPLICATION

(11) **EP 4 140 407 A1**
(43) Date of publication of application: **01.03.2023**
(21) Application number: 22192865.8
(22) Date of filing: 30.08.2022
(51) Int. Cl.: A61B 5/11, A61B 5/00

(54) **A METHOD AND A DEVICE FOR RETRIEVING SENSOR DATA FOR MEDICAL MOTION RECOGNITION**

(30) Priority: 30.08.2021 EP 21193834
(71) Applicant: Gaia AG, 22085 Hamburg (DE)
(72) Inventor: Weiss, Mario, 22085 Hamburg (DE)
(74) Representative: RGTH

(57) **Abstract**

The present invention relates to a method for retrieving sensor data for medical motion recognition, wherein the method comprises retrieving and recording data from multiple motion sensors on a person for a period of time of at least 24 hours, and wherein the method further comprises displaying at least time segments of the retrieved and recorded data in the form of an avatar for visualizing movements of the person within the time segment.

## Description

The present invention relates to a method for retrieving sensor data for medical motion recognition as well as to a respective device according to the independent claims.

### Prior art

Epilepsy is one of the most common neurological diseases worldwide. The disease is mainly characterized by unpredictable, spontaneous epileptic seizures, which can be treated medically. Depending on the type of epilepsy, those affected can lead an almost carefree life without major restrictions on their autonomy. However, a central part of the treatment is the suppression of seizures with medication wherein the success of the treatment, namely the reduction in the frequency and severity of the seizures, is determined by the correct identification of the type, amount, and frequency of the seizures. This is typically done by a mixture of diagnostic procedures.

A cornerstone of the diagnostic process is the identification of numbers and especially the expressions of seizures by asking patients and/or relatives to recall events. As this method is highly subjective, the perfect situation is that the physician can actually see a seizure. As seizures might happen only once a week or a month, physicians rely on 24/7 video capturing that is normally done in a hospital setting where patients have to stay for 2 to 3 weeks. ECG is also an important element of the diagnostic process, but not all electrical misfunctions lead to clinically relevant misfunctions, so that it can be misleading. Capturing and actually being able to see and interpret a seizure of a patient is most critical for the specialist physician. This analysis then leads to a highly differentiated patient-specific treatment plan.

### Summary of the invention

The objective of the present invention is to provide an effective visualization monitoring of a person, which on the one hand allows a medical motion recognition and potentially a diagnosis of the person and which, on the other hand, can easily be integrated into the person's everyday life. The person is, in particular, a patient.

The present invention relates to a method for retrieving sensor data for medical motion recognition which comprises retrieving and recording data from multiple motion sensors which are arranged on a person for a period of time of at least 24 hours. The method further comprises displaying at least time segments of the retrieved and recorded data in the form of an avatar for visualizing the movements of the person within the time segment. In particular, more than five, especially more than ten or more than thirteen sensors are used. The terms movement and motion are used as synonyms.

The method is particularly a computer-implemented method and allows an effective and reliable collection of sensor data for medical motion recognition which at the same time does not prevent the person from following his or her everyday life. In particular, the data is retrieved and recorded for a period of time of at least 72 hours. In particular, the data can be retrieved and recorded for a longer period of time, such as, for example a week. The long duration of the data retrieval is crucial for detecting almost coincidental events such as e.g., an epileptic seizure.

The multiple motion sensors represent in particular a sensor system that is installed on the person. In particular, the method can comprise installing said motion sensors on the person at multiple different locations. In particular, the sensors are installed at extremities such as for example limbs of the person. There can be at least one sensor installed at each extremity of the person. A sensor can be installed at each hand and/or each forearm and/or each upper arm and/or each thigh and/or each lower leg. In addition, there can be at least one sensor at the torso of the person. The sensors serve to sense the movement of the body part at which they are disposed. The sensors can be located directly on the body; thus, they can be placed on the skin of the person. They can be attached, e.g., via an adhesive. Further, they can be embedded into sleeves or clothing which the person wears.

In particular, the sensors are each three-axis motion sensors, in other words, accelerometer sensors, which detect an orientation as well as a change in velocity, thus an acceleration, e.g., based on a translational or rotational movement, of the body part at which they are installed. Further, the sensors can be adapted to sense a magnetic field at the particular location of the sensor. In addition, the sensor can comprise a gyroscope.

The sensor especially retrieves data regularly, especially with a frequency of at least 20 Hz, more particularly at least 25 Hz, and more preferred at least 30 Hz. Further, the frequency can be at least 50 Hz, even more preferred at least 70 Hz, and most preferred at least 90 Hz. All data retrieved by a sensor can be understood as sensor data.

In particular, the sensors can each be a multimodal sensor that can not only retrieve motion data but for example, can also retrieve other medical information such as for example EGK, sEMG, EEG, electrodermal activity, or blood glucose levels. In particular, the method does not consider sensor data which is from a video detection system.

The method can comprise a fusion of the sensor data of the employed sensors. In other words, the sensor data is combined. Part of the fusion can be synchronization and denoising of the data. Further, redundant data can be recognized and discarded.

Especially, an absolute three-dimensional orientation of each sensor can be determined. For example, a Quaternion or Euler angles for each sensor can be determined. This is especially done with the same frequency as the data retrieval. In other words, every time data is retrieved, the orientation is determined. Especially, a time stamp is assigned to each determined orientation.

The determination of the orientation and preferably the assignment of a time stamp can be done in an orientation determination unit which is the most preferred part of each sensor. In other words, each sensor can comprise an orientation determination unit such that the orientation is determined locally, not centrally, and preferably also stored locally before it is transmitted. The fact that the data can be stored locally is especially advantageous for possible problems in the transmission to the assessment unit. For example, there can be problems in the wireless or Bluetooth connection, which do not affect the data retrieval since the data can be temporarily stored on the sensors.

If the time stamp is assigned before the transmission, thus on the sensor, the time stamp is extremely accurate, which can be advantageous for precise synchronization. If the time stamp is not assigned at the sensor, it can later be assigned in the assessment unit.

The orientations and preferably the time stamps can then be transmitted to an assessment unit which can be located on a mobile device, such as a smartphone or tablet. If a real-time display is to be achieved, the orientations can be transmitted without or before storing them locally on the sensor. Alternatively, or additionally, the unanalyzed sensor data, in other words, the raw data, can be transmitted.

Furthermore, the method can comprise a mapping of the sensor data or the orientations to a body part of the person. Thus, the method can identify the movement of each body part at which a sensor is installed. The mapping is preferably based on pattern recognition. Thus, the method can classify the location of each sensor based on the sensor data, the determined orientations, and the movement patterns, which are compared to predefined movement patterns assigned to a body part. Especially, disease-specific movement patterns are predefined. In addition, movement patterns of activities of daily living can be pre-defined so that movement related to activities of daily living can be identified and differentiated from disease-specific movements. Preferably, the method comprises identifying disease-specific movements patterns by comparing those with the pre-defined patterns. The method can comprise labelling the recognized movement pattern as disease-specific if the comparison shows that the highest similarity is with a disease-specific pre-defined pattern. In particular, only disease-specific movement patterns are predefined.

By determining the absolute orientations over time, the movement of the body part to which a sensor is mapped can be displayed. The method comprises displaying at least time segments of the retrieved and recorded data in the form of an avatar. An avatar is a graphical representation of the person. In particular, the avatar represents not only a body part of the person but the entire body so that sensor data of all motion sensors are used to represent the movements of the person. In particular, the avatar preferably has a two-dimensional form or a three-dimensional form.

The avatar can be comprised of body segments wherein, at least on a part of the body segments, a sensor is installed. Thus, the movement, as well as the orientation of the respective body segment, can easily be determined and displayed. By determining the orientation of all body parts at which a sensor is installed, the person's movements can be displayed as the avatar.

The method can especially comprise displaying the data in the form of an avatar on a screen for a medical professional to be seen in order to assess the motions, in other words, movements, of the person. Thus, even though the person had not been monitored via video and could pursue his everyday life, at least time segments, preferably all of the recorded data, can be shown to a medical professional in order to evaluate and assess the motions. The present invention makes it possible to recognize motions that could be medically relevant in a setting that is transferred from the context of the doctor's office or a clinic directly to the everyday life of the person such that the person has minimum restrictions while being monitored. While the present invention thus allows the person to have the least restrictions to his everyday life, it allows an effective diagnosis and monitoring of the success of treatment of the person. Especially, displaying the data can be conducted in real-time, which is enabled by the high frequency of sensor data retrieval, sensor fusion, and precise synchronization. The method allows for further enhancement of the display, such as e.g., fast forward replay, pause, or slow-motion function. Further, the size of the avatar, in other words, the distance at which the avatar is shown, in other words, a zoom function, and the perspective, namely the angle at which a view on the avatar is displayed, can be amended.

The main challenge is to enable a method or device to differentiate seizures from normal, daily living, in particular repetitive, movements, in other words from movement pattern related to activities of daily living. Movement patterns can be based on repetitive movements such as e.g., breathing.

Thus, the method especially comprises evaluating the retrieved and recorded data for classifying at least one time segment of the data as relevant by an expert. In particular, the method classifies certain time segments of the data as relevant, while other time segments are classified as irrelevant. Relevant would thus relate to medically significant motions, such as e.g., patient and/or disease-specific movement patterns, wherein irrelevant time segments would relate to everyday life, in other words, activities of daily living that could be repetitive or non-repetitive. Further, the activities of daily living can generate motion patterns that could be misleadingly classified as disease-specific. Especially, relevant time segments can be stored, e.g., for later further analysis. In particular, only relevant time segments are stored.

Classifying means identifying time segments that are similar to a predefined motion pattern. In particular, the motion pattern refers to, e.g., repetitive or disease - characteristic movements of at least one or multiple body parts.

By means of the sensors, a movement of at least a body part can be detected. A time segment can be assigned to the movement in which the movement took place. The method can preselect time segments with movements, especially disease-specific movements, for evaluation. In particular, the evaluation comprises comparing the data, in other words, the detected movements, with at least one predefined motion pattern and determining a respective correlation between the data and the at least one motion pattern. In particular, the evaluation comprises comparing time segments of the data with the at least predefined motion pattern and classifying each time segment. In this regard, the data of each sensor can be evaluated individually or evaluated collectively.

Especially when evaluating the sensor data collectively, the method can determine a correlation between motions of different sensors at the same time. For example, it is important to recognize whether a repetitive motion is detected by multiple sensors at the same time. Furthermore, the locations of the sensors which detected the same motion pattern can contribute to the evaluation. In this regard, a cross-correlation between the motions detected by different sensors can be determined. Apart from repetitive motions, disease-specific motions, e.g., the drift of a person's head while sleeping, can be detected. Each above step is preferably carried out for all predefined motion patterns. In particular, the method comprises identifying the motion pattern regarding which the correlation is the highest as compared to other motion patterns. This motion pattern can then be assigned to the time segment.

By determining a respective correlation between the data and at least one preferably clinically relevant motion pattern, it is determined how similar the detected motion of the data is to a predefined motion pattern. The higher the correlation is, the more similar the data is to at least one motion pattern. This also helps the physician to "tag" specific movement patterns. The method can include searching for similar movements of a movement that has been tagged for speeding up the screening process.

The comparison can relate to a frequency and/or an amplitude and/or a duration of the detected motion and the at least one motion pattern. For example, the method can determine the frequency, especially a fundamental frequency, of a detected movement. Furthermore, the method can determine an amplitude, for example, an average amplitude, of the repetitive movement. These data can be compared to at least one predefined motion pattern. Especially regarding the time duration, the method can include defining a time minimum. The time minimum can, e.g., be one minute. Only repetitive movements being longer than the time limit can be evaluated.

It is possible that a detected movement is a superimposition of different movement components with different frequencies. Thus, before the actual comparison, lowfrequency components of the data can be removed which are unrelated to seizure activities. For this purpose, a frequency threshold for removing rhythmic movement components with a lower frequency can be pre-defined. The remaining higher frequency movement components can be compared as explained above, e.g., regarding their respective amplitude, frequency and/or duration.

Especially, time segments are classified as relevant when the correlation assigned to the time segment exceeds a predefined threshold. In other words, the method can comprise defining a threshold regarding the similarity between a recognized motion and a predefined motion pattern, and depending on the comparison with the threshold, the method can comprise classifying time segments as relevant or irrelevant. Relevant time segments can then be labeled as relevant, and others can be labeled as irrelevant. The decision of whether a time segment is classified as relevant or not can also be influenced by which motion pattern has been assigned to the time segment and whether this motion pattern has been pre-defined as clinically relevant. Thus, if the threshold of the correlation to a predefined clinically relevant motion pattern is exceeded, the time segment can be classified as relevant. The physician can look at the movement of the avatar and accept or relabel the relevance of the related time segment. This then leads to an optimization of the decision patterns.

At least one motion pattern can relate to a seizure, in particular an epileptic seizure. Since there are different types of seizures, in particular epileptic seizures, multiple different motion patterns can be predefined. Within the pre-defined patterns, the number of involved sensors which detect the same or at least a similar movement can be different; furthermore, the intensity (in other words, amplitude) or frequency, as well as the time duration, can differ.

The above-described evaluation can preferably be done by a neural network that is trained with training data to recognize at least one motion pattern. In particular, the neural network is trained on multiple data sets from motion sensors representing different motion patterns, such for example, different, preferably epileptic, seizures. By providing the network with a manually derived respective labeling of whether the motion is relevant or not relevant, or even more preferred, which type of seizure has occurred, the neural network can learn to recognize a motion consequently and efficiently.

Further, the method can include labeling relevant time segments as a seizure and nonrelevant time segments as no seizure. However, this labeling is not a diagnosis; it is preassessment to help a medical professional to find potentially relevant time segments faster and then evaluate the data. Furthermore, the method could include labeling the seizures in more detail, for example, indicating the type of the seizure. In this regard, the method can indicate, for example, the seizure type with which the motion of a particular time segment has the most similarity.

The method can comprise displaying only relevant time segments of retrieved and recorded data to a medical professional for a diagnosis of a disease of the person.

Since the method can classify time segments of the recorded data, a medical professional can much more easily and faster diagnose a person since the professional does not have to watch all the detected motions which occurred during the monitoring time but can only look at relevant time segments of it.

As explained above, the diagnosis, as well as treatment of epilepsy, is challenging. While the present invention does not only achieve collecting data for an initial diagnosis of epilepsy, it also allows constant monitoring of the person afterward to evaluate whether a treatment is successful in reducing the number and quality of occurring seizures. The invention thus improves the detection of clinically relevant seizures and its documentation for providing more detailed information to a medical professional for diagnosing the person with a disease and/or for evaluating whether treatment of a disease is successful. In other words, the present invention provides automatic recognition of seizures. Because it is also possible to collect additional data on heart rates, glucose levels, or brain activity, it is advantageous to determine further correlations between the additional data and the detected movements, which can guide the therapeutic decision process further.

Furthermore, the method could be used for objective progression documentation and measurement of other neurological diseases than epilepsy, such as for example multiple sclerosis, Parkinson's disease, or apoplexy. The method could be used for monitoring the progression of neurological disease by collecting sensor data, as explained above. For example, it could be evaluated how the range of movements of the person is affected by a particular disease. Thus, the progression of a disease could be evaluated at the same time. Further, it could be evaluated how well the person responds to treatment, as explained above.

Another possible use of the method is to monitor a movement of a person in a sports or physical exercise context, for example, doing particular exercises or training particular movements, e.g., during physical therapy. Since the method is configured to collect the data and to represent it in the form of an avatar, a trainer or the person itself can easily and effectively monitor the movements for training purposes. In this context, a long duration of the sensor retrieval is important since changes in a movement or posture of a person occur over a longer period of time.

In addition, the method can comprise providing feedback to the person, especially in real-time. For example, the method can comprise detecting a disadvantageous movement or posture. For example, if the person is to conduct a particular exercise, the method can, in a first step, identify the exercise, preferably by pattern recognition, and then recognize whether the movement is conducted correctly. If there is a discrepancy, feedback can be provided. Before, the method comprised classifying a discrepancy between the correct movement and the actual movement and only if a pre-defined threshold is exceeded.

The feedback can be visual, auditive, or haptic and be provided by a mobile device connected to the sensors. The same can apply to a person trying to maintain a better posture. If the person falls back into an unhealthy posture, feedback can be provided. In addition, the discrepancies in the correct movement or posture can be stored for later analysis, e.g., to evaluate an improvement during physical therapy.

Furthermore, the sensors can comprise counting the steps of a person. In addition, the distance that a person has covered can be determined.

The method can comprise identifying accidentally dropped sensors by recognizing abnormalities of the determined orientations.

All the above features relating to the movements of a person can also relate to the movements of an animal, such as e.g., a horse.

In another aspect of the invention, it relates to a device for retrieving sensor data for medical motion recognition wherein the device is configured to conduct an above-described method. In particular, the device can be configured to conduct each above-described step. In particular, the device includes multiple sensors for retrieving motion data.

Each sensor can have a water-resistant casing. Especially, the casings have no sockets for connecting the respective sensor to other devices, e.g., to charge the sensor. The sensors can be updated wirelessly "over-the-air" and also charged wirelessly. The configuration of the casings enables electrical touch protection. Further, the person wearing the sensors is the least restricted in their daily activities, which can include contact with water, such as e.g., swimming or taking showers. In addition, the configuration allows using the sensors for monitoring the movements of a person during swimming which would otherwise not be possible. In addition, the casing can be removable, which allows a replacement of the battery of a sensor. Overall, the sensor is configured in the most sustainable way, since by e.g., replacing the batteries and the casing, the sensor can be easily refurbished.

Furthermore, the device can include a storage unit, such as e.g., a memory, to record the sensor data and a display unit to display the retrieved and recorded data in the form of the avatar. Each sensor can have a local storage unit. Further, there can be a central storage unit. The display unit of the device can e.g., be a screen of any mobile device, such as a smartphone or tablet.

Especially each sensor can comprise an orientation determination unit for determining the absolute orientation and possibly assigning a time stamp before transmitting it. The data, either the unanalyzed sensor data or the orientations of the different sensors, can preferably be transmitted, for example, via Bluetooth or WIFI, to a central storage unit, which could be configured as a cloud. Furthermore, the device can comprise an assessment unit for the above-described evaluation. The data can be transmitted to the assessment unit, which can, e.g., conduct the steps of evaluating the retrieved and recorded data, identifying relevant time segments to display only relevant time segments. The storage unit records the results of the evaluation, such as motion patterns, date, time, duration, frequency, and amplitude of detected motions of each sensor.

The importance is that multiple sensors are used so that each preferably epileptic seizure can be monitored and detected. For example, using only a wrist band including a motion sensor is not sufficient since, for example, a person can have a pathological movement in the legs as well as body rocking, but no rhythmic arm movements, and therefore a respective seizure would not be detected by the respective wrist band. The device can, in particular, be configured to display the data for the entire period of time or, depending on the evaluation, only display relevant time segments. The device is in particular a seizure recognition system for automated seizure recognition.

Especially, the device is configured to retrieve and record data from multiple motion sensors on a person for a period of time of at least 24 hours, wherein the device is further configured to display at least time segments of the retrieved and recorded data in the form of an avatar for visualizing movements of the person within the time segment.

In particular, the device is configured to retrieve and record for a period of time of at least 72 hours.

Further preferred, the device can be configured to evaluate the retrieved and recorded data for classifying at least one time segment of the data as relevant.

The device can be configured to compare the data with at least one predefined motion pattern and determine a respective correlation between the data and the at least one motion pattern. The comparison can relate to a frequency and/or an amplitude of a detected motion and the at least one motion pattern.

The device can be configured to classify time segments of the data as relevant if the correlation assigned to the time segment exceeds a predefined threshold. Especially, at least one motion pattern relates to a seizure.

The device can comprise a neural network configured to conduct the evaluation;
said neural network is trained with training data to recognize at least one motion pattern.

The device can be configured to display only relevant time segments of retrieved and recorded data to a health professional for a diagnosis of a disease of a person or for monitoring of a disease of a person.

Furthermore, the invention relates to a computer program product, comprising a computer-readable store medium in which a program is stored which, after being loaded into the memory of a computer, enables the computer to carry out an above-described method in corporation with an above-described device.

In addition, the invention relates to a computer-readable store medium on which a program is stored, which, after being loaded into the memory of the computer, enables the computer to carry out an above-described method in corporation with an above-described device.

### Brief description of the drawings

The above-mentioned and other features of the invention disclosed herein are described below with reference to the drawings of the preferred embodiments. The illustrated embodiments are intended to illustrate, but not to limit, the invention. The drawings contain the following features:
- Figure 1: a schematic flow chart of a method according to the invention;
- Figure 2: a schematic overview of a device according to the invention; and
- Figure 3: an avatar derived from the sensor data.

### Detailed description of the preferred embodiments

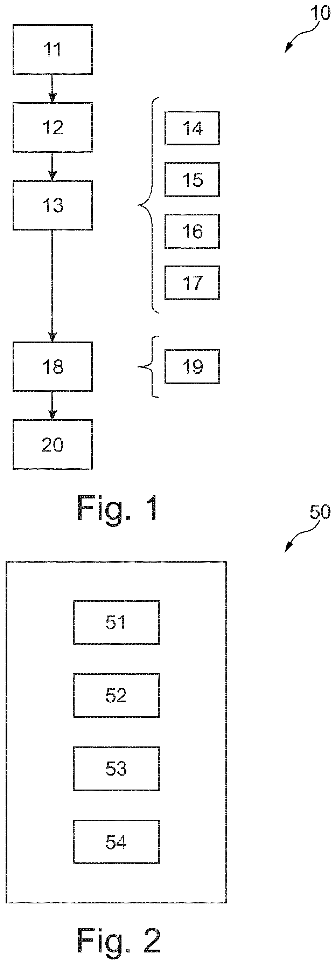

Figure 1 shows a schematic flow chart of a method 10 according to the invention for retrieving sensor data for medical motion recognition.

The method 10 can comprise installing 11 said motion sensors on the person on multiple different locations. Based on the method 10 data is retrieved and recorded 12 from the multiple motion sensors and at least time segments of the retrieved data and recorded data are displayed 18 in the form of an avatar. In this way movements of the person are visualized 20.

Before the data is displayed, it can be evaluated 13. In particular, the data is compared 14 with at least one predefined motion pattern. In the scope of the comparison 14 a respective correlation between the data and at least one motion pattern can be determined 15. The time segments of the data can then be classified 16 as relevant if the correlation assigned to the respective time segment exceeds a predefined threshold. These time segments can then be labeled 17 as relevant. In this way, only relevant time segments of retrieved and recorded data can be displayed 19.

Figure 2 shows a schematic overview of a device 50 according to the invention which comprises the motion sensors 51 which are disposed at different parts of the body of a person, and a storage unit 52 to record the respective data. There can be a storage unit 52 assigned to each motion sensor 51 and disposed at the sensor 51 directly. In addition, or alternatively, there can be a central storage unit 52. Each sensor 51 or its storage unit 52 can be configured to communicate with the central storage unit 52 to record the retrieved data or to transmit recorded data.

The device 50 further comprises a display unit 53 to display at least time segments of the retrieved and recorded data in the form of an avatar representing the person. Furthermore, the device 50 can comprise an assessment unit 54, which is configured to evaluate the retrieved and recorded data, especially to identify relevant time segments and thus only display relevant time segments of the retrieved and recorded data.

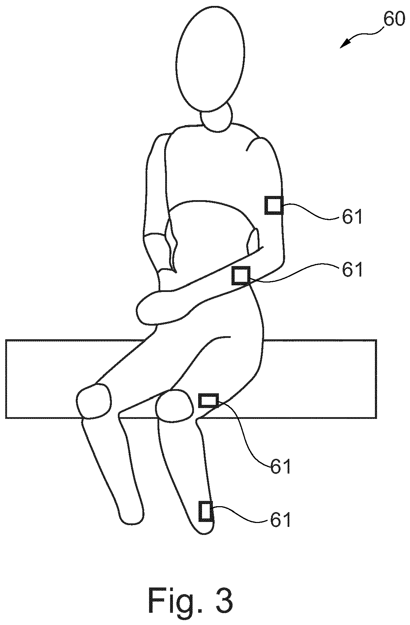

Figure 3 shows an avatar that is derived from the data of the sensors 51. The avatar 61 comprises body segments 61. The movements of the avatar are derived from the sensors 51, which are installed at the respective body parts.

### Reference signs

- 10: method for retrieving sensor data for medical motion recognition
- 11: installing said motion sensors on the person on multiple different locations
- 12: retrieving and recording data from multiple motion sensors
- 13: evaluating the retrieved and recorded data
- 14: comparing the data with at least one predefined motion pattern
- 15: determining a respective correlation between the data and the at least one motion pattern
- 16: classifying at least one time segment of the data as relevant
- 17: labeling time segments of the data as relevant
- 18: displaying at least time segments of the retrieved and recorded data in the form of an avatar
- 19: displaying only relevant time segments of retrieved and recorded data
- 20: visualizing movements of the person within the time segment

- 50: device
- 51: motions sensors
- 52: storage unit
- 53: display unit
- 54: assessment unit

- 60: avatar
- 61: body segment

## Claims

1. A method for retrieving sensor data for medical motion recognition,
**characterized in that**
the method comprises retrieving and recording data from multiple motion sensors on a person for a period of time of at least 24 hours,
wherein the method further comprises displaying at least time segments of the retrieved and recorded data in the form of an avatar for visualizing movements of the person within the time segment.

2. The method according to claim 1,
**characterized in that**
the data is retrieved and recorded for a period of time of at least 72 hours.

3. The method according any one of claims 1 or 2,
**characterized in that**
the method comprises installing said motion sensors on the person on multiple different locations.

4. The method according any one of preceding claims,
**characterized in that**
the method comprises evaluating the retrieved and recorded data for classifying at least one time segment of the data as relevant.

5. The method according to claim 4,
**characterized in that**
the evaluation comprises comparing the data with at least one predefined motion pattern and determining a respective correlation between the data and the at least one motion pattern.

6. The method according to claim 5,
**characterized in that**
the comparison relates to a frequency and/or an amplitude of a detected motion and the at least one motion pattern.

7. The method according to claim 5 or 6,
**characterized in that**
the method comprises classifying time segments of the data as relevant if the correlation assigned to the time segment exceeds a predefined threshold.

8. The method according to any one of claims 5 to 7,
**characterized in that**
the at least one motion pattern relates to a seizure.

9. The method according to any one of claims 4 to 8,
**characterized in that**
the evaluation is done by a neural network,
said neural network being trained with training data to recognize at least one motion pattern.

10. The method according to any one of claims 4 to 9,
**characterized in that**
the method comprises displaying only relevant time segments of retrieved and recorded data to a health professional for a diagnosis of a disease of the person or for monitoring of a disease of a person.

11. A device for retrieving sensor data for medical motion recognition,
**characterized in that**
the device is configured to conduct a method according to any one of claims 1 to 10.

12. A computer program product, comprising a computer-readable storage medium on which a program is stored, which, after being loaded into the memory of a computer, enables the computer to carry out a method according to any one of claims 1 to 10 in cooperation with a device according to claim 11.

13. A computer-readable storage medium on which a program is stored, which, after being loaded into the memory of a computer, enables the computer to carry out a method according to any one of claims 1 to 10 in cooperation with a device according to claim 11.

## Amended claims

### Amended claims in accordance with Rule 137(2) EPC.

1. A method for retrieving sensor data for medical motion recognition, **characterized in that**
the method comprises retrieving and recording data from multiple motion sensors on a person for a period of time of at least 24 hours,
wherein the method further comprises displaying at least time segments of the retrieved and recorded data in the form of an avatar for visualizing movements of the person within the time segment,
wherein the method comprises evaluating the retrieved and recorded data for classifying at least one time segment of the data as relevant,
wherein the evaluation comprises comparing the data with at least one predefined motion pattern and determining a respective correlation between the data and the at least one motion pattern, and
wherein the method comprises classifying time segments of the data as relevant if the correlation assigned to the time segment exceeds a predefined threshold.

2. The method according to claim 1,
**characterized in that**
the data is retrieved and recorded for a period of time of at least 72 hours.

3. The method according any one of claims 1 or 2,
**characterized in that**
the method comprises installing said motion sensors on the person on multiple different locations.

4. The method according to claim 1,
**characterized in that**
the comparison relates to a frequency and/or an amplitude of a detected motion and the at least one motion pattern.

5. The method according to any one of claims 1 or 4,
**characterized in that**
the at least one motion pattern relates to a seizure.

6. The method according to any one of claims 1, 4 or 5,
**characterized in that**
the evaluation is done by a neural network,
said neural network being trained with training data to recognize at least one motion pattern.

7. The method according to any one of claims 1, 4, 5 or 6,
**characterized in that**
the method comprises displaying only relevant time segments of retrieved and recorded data to a health professional for a diagnosis of a disease of the person or for monitoring of a disease of a person.

8. A device for retrieving sensor data for medical motion recognition,
**characterized in that**
the device is configured to conduct a method according to any one of claims 1 to 7.

9. A computer program product, comprising a computer-readable storage medium on which a program is stored, which, after being loaded into the memory of a computer, enables the computer to carry out a method according to any one of claims 1 to 7 in cooperation with a device according to claim 8.

10. A computer-readable storage medium on which a program is stored, which, after being loaded into the memory of a computer, enables the computer to carry out a method according to any one of claims 1 to 7 in cooperation with a device according to claim 8.
